# EUROPEAN PATENT APPLICATION

(11) **EP 4 797 190 A1**
(43) Date of publication of application: **26.08.2026**
(21) Application number: 25159098.0
(22) Date of filing: 20.02.2025
(51) Int. Cl.: G06Q 50/02, A23K 10/30, A23K 50/00, A23L 5/00, G01N 33/02, G06N 20/00, G09B 19/00, G09B 23/36, G16C 20/30, G16C 20/70

(54) **FOODSTUFF COMPOSITION ESTIMATION METHOD**

(71) Applicant: Quanturi Oy, 00260 Helsinki (FI)
(72) Inventor: Pesonen, Nadine, 00350 Helsinki (FI); Huyghe, Marie, 00370 Helsinki (FI); Raju, Geet, 00560 Helsinki (FI)
(74) Representative: Kolster Oy Ab

(57) **Abstract**

The present disclosure relates to a method of estimating a composition of a foodstuff comprising: providing a plurality of input variables, the input variables relating to the growing and/or processing conditions of the foodstuff. The method comprises providing a computational model comprising data relating to the input variables and data relating to a known composition of a foodstuff associated with the input variables and receiving a value for a plurality of input variables and estimating the composition of the foodstuff in accordance with said input variable values using said computational model.

## Description

The present disclosure relates to a method of estimating a composition of a foodstuff (e.g. animal fodder).

### Background of the Invention

Fodder is agricultural foodstuffs for domesticated livestock such as cattle, horses, goats, sheep, pig, rabbits and chickens. Fodder can be a mixture of grass, legumes and other herbaceous plants that might be region specific. Fodder is typically cut and harvested from crops and given to animals or stored for a period of time before feeding it to the animals. Fodder is a critical diet compound of all grazing domesticated livestock as it constitutes a complementary element or even a full substitute to the required animal feed. Fodder is fed to livestock to increase the quality and consistency of the feed, or to replace grazing when cattle is permanently kept in stables or during times when pastures are unavailable due to factors like winter, droughts, or scattered fields.

The quality of fodder is paramount for animals to reach greater production and improved efficiency (pounds of milk or weight gained per pound of feed consumed) as well as improved animal wellbeing. Caloric demands vary based on the animal's life stage or purpose (growing, lactating, dry, draft horses, competition horses, etc.). Most of the caloric requirements are met by the composition of fodder. Therefore, the composition of fodder has a fundamental influence on productivity, health, and welfare of the animal. Fodder composition also affects animal product quality and safety, and the environment.

Traditionally, fodder composition is determined through laboratory analysis of fodder samples after harvest. In developed countries, it is common practice to undertake most of the analyses in accredited laboratories. These analyses involve either wet chemistry to quantify protein, fibre, fat, and minerals or near-infrared reflectance spectroscopy (NIR) to measure nutrient content.

The inventor has found numerous problems with prior art methods. The chemical method typically takes three weeks to one month, while the near infrared method could take up to one week in a laboratory or provide immediate results using a portable analyser. A typical laboratory analysis reports the composition of fodder, which then defines its quality. Sampling is limited in scale and frequency and is sporadic due to manual labour requirements and associated costs. Prior to cutting and harvest, recommendations based on historical weather data (e.g. Growing Degree Days (GDD) or Dew points) devised by local or national authorities, associations or lookup tables help define the most appropriate cutting timeframe for reaching higher quality fodder. These are only generic recommendations that primarily apply to the first cutting of the plants after vegetative dormancy and cannot tell the precise composition of fodder after harvest.

The present invention aims to overcome or ameliorate one or more of the above problems.

### Statement of Invention

According to a first aspect, there is provided: a method of estimating a composition of a foodstuff comprising: providing a plurality of input variables, the input variables relating to the growing and/or processing conditions of the foodstuff; providing a computational model comprising data relating to the input variables and data relating to a known composition of a foodstuff associated with the input variables; and receiving a value for a plurality of input variables and estimating the composition of the foodstuff in accordance with said input variable values using said computational model.

The method may comprise inputting data relating to the input variables and data relating to the known composition of a foodstuff associated with the input variables to train said computational model.

The growing and/or processing conditions may comprise one or more of: a geographic location of foodstuff during growth thereof; a coverage rate of the foodstuff; a species or variety of the foodstuff; a cutting time or timeline of the foodstuff; a cutting frequency of the foodstuff; a processing state of the foodstuff; farming practices for the foodstuff; soil composition in which the foodstuff is grown; condition of the foodstuff after processing; or weather condition curing processing or growth of the foodstuff. The growing conditions may comprise environmental in which the foodstuff was grown.

The foodstuff composition may comprise a nutritional value of the foodstuff. The nutritional value of the foodstuff may comprise one or more of: a protein content; a carbohydrate content; fibre content; moisture content; or mineral/vitamin content. The method may comprise estimating one or more nutritional value independently of one or more further nutritional value.

The foodstuff may comprise a crop or processed product thereof. The crop may comprise one or more of: grass; legumes; cereal; or clover.

The composition of the foodstuff may be estimated after harvest and/or subsequent processing thereof. The foodstuff may comprise an animal feed (e.g. fodder). The composition of the foodstuff may be estimated before being used as fodder. The foodstuff may comprise one or more of: hay; silage; haylage; straw; sprouted grains; legumes; or pellets of said crops.

The computational model may comprise a neural network or machine learning model.

A laboratory composition value for a given set of values for the input variables is input into the computational model. The values are compared to the corresponding estimated composition data associated with said set of values for the input variables to further train said computational model (i.e. to provide feedback).

The input variable data and/or values are received from a remote database. The remote database may comprise a government database. The input variable data and/or values may be retrieved via an API. The input variable data and/or values may be automatically retrieved.

Input variable data and/or values may be received from an agricultural processing and/or harvesting machine (e.g. a combine harvester). Input variable data and/or values may be determined from aerial/satellite imagery.

The method may comprise a plurality of users. Each user may be able to estimate the composition. A common computational model may be used to provide said estimate the users. Input data may be provided by two or more of the users to train the computational model.

According to a second aspect, there is provided: a computer program or computer readable medium comprising program instructions which, when executed by the computer, cause the computer to carry out a computer process implementing the method according to any preceding claim.

According to a third aspect, there is provided: a computer system configured to estimate a composition of a foodstuff comprising a controller configured to: provide a plurality of input variables, the input variables relating to the growing and/or processing conditions of the foodstuff; provide a computational model comprising data relating to the input variables and data relating to the known composition of a foodstuff associated with the input variables; and receive a value for a plurality of input variables and estimate the composition of the foodstuff in accordance with said input variable values using said computational model.

The computer may be a remote device. The computer may operatively communicate with a user device. The user may input variable values via the user device. The computer may be configured to operatively communicate with one or more remote database. The computer may comprise a processer, memory, GPU and/or a communication interface.

Any aspect of the invention may be combined with any other aspect of the invention where practicable.

### Description

Embodiments of the present invention are described below, by way of example only, with reference to the accompanying drawings:
**Figure 1** shows a schematic view of a fodder production method;
**Figure 2** shows a schematic view of fodder composition estimation method;
**Figure 3** shows a first schematic view of a fodder composition estimation system;
**Figure 4** shows a second schematic view of a fodder composition estimation system;
**Figure 5** shows a first graph of an estimated composition versus and laboratory measured composition;
**Figure 6** shows a second graph of an estimated composition versus and laboratory measured composition.

The present method provides a method to determine or estimate a composition (e.g. a nutritional value) of a fodder. The composition of the fodder is determined prior to immediate feeding or storage of the fodder, such that the nutritional composition of the fodder fed to animals can be determined accordingly.

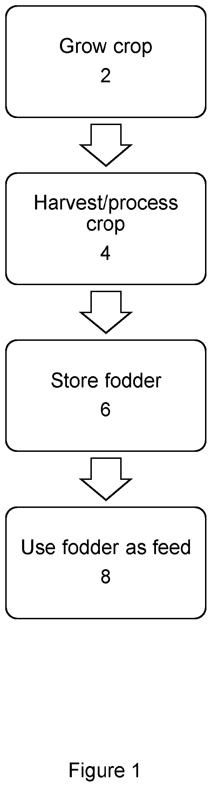

The initial stages of the process are described with reference to figure 1. In a first step 2, the crop is grown. The crop may comprise a grass and/or legume. The crop may be grown in a field containing both a grass and legume, or a field comprising only a grass or a legume. The crop may comprise one or more of, inter alia: ryegrass; timothy; brome; fescue; Bermuda grass; orchard grass; alfalfa (Lucerne); clover (red, white and/or subterranean); cereals (e.g. grains); any other herbaceous plant or, grass or legume; and/or combinations thereof.

In second step 4, the crop is then harvested. Typically, the crop is fully or partially cut and harvested in a conventional manner. Further processing to the crop may be provided, for example, one or more of: raking; tedding; baling; chopping; drying; dehydration; compaction; and wrapping. The processed crop may provide one or more of: hay; silage; haylage; straw; sprouted grains; legumes; oils; or pellets of said crops. The harvested and/or processed crop provides the fodder accordingly.

In step 6, the fodder may be stored for later use. Storage of the fodder may comprise: storage in warehouses, bunkers, silos etc; open air; under a tarpaulin or cover; or in wrapping (e.g. in silage wrap). Fodder may be stored in countable/discrete units (e.g. bales or the like) or stacked in bulk.

In a final step 8, the fodder is used and fed to animals. In some embodiments, the fodder may be fed to animals without storage (i.e. directly after processing).

It can be understood that the process described with reference to figure 1 conventional and not pertinent to the invention at hand.

Before feeding of the fodder to animals the composition of the fodder is estimated. This allows the user to determine the quality of the fodder. This allows the user to determine how much fodder is used and/or whether the fodder requires supplementing with other materials (e.g. other fodder or supplements). Composition of the fodder is estimated using a computational model. Various variables relating to the crops/fodder are input into the model along with known values for the composition with said values. Thus, a model of the composition of the fodder in accordance with said variables is constructed. The user can therefore input their own values for some or all the variables and estimate the composition of the fodder without the need to conduct expensive and/or lengthy analytical tests.

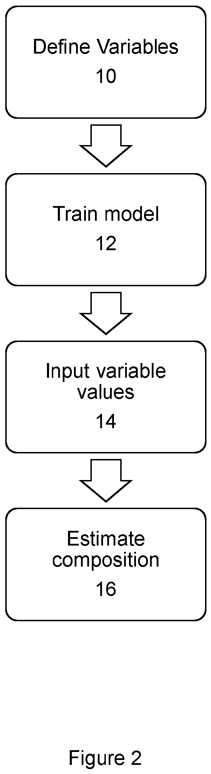

The process of constructing and using the computational model is shown in detail in figure 2. In a first step 10, the variables of the system are defined to construct the computational model. The input variables are defined. Typically, the input variables relate to the environmental condition during growth of the crop/fodder and/or processing conditions thereof. The input variables comprise one or more of:
- A geographical location of the crop. The location may comprise coordinates (e.g. latitude and longitude) of the crop. The location may define a zone, boundary or area of the crop. The location may be granular (e.g. limited to discrete values of latitude and longitude or regions of a country etc.) or may be high accuracy (e.g. down to 1m or 10m accuracy). Location data may be obtained via any suitable means, inter alia: satellite/aerial imagery; cellular networks; localised geo-positioning sensors (e.g. GPS sensors); digital mapping services; authority (e.g. government or government agency) registries; or manual input. Location data may be collected for example, via a user's mobile (cellular) phone or tracking systems in agricultural machinery.
- Crop coverage. This is the amount of area the crop covers relative to the geographic area the crop is contained. This may be provided as a percentage or fraction of the total area. The crop coverage may be determined in a similar manner to the geographical area. The crop coverage may be determined from the yield collected from a specific area (e.g. the yield determined during harvest thereof via an agricultural machine).
- The plant species or variety of the crop. The species/variety may be obtained one or more of: authority (e.g. government or government agency) registries; national or producer databases; laboratory analysis; or manual input (e.g. when the user simply knows the species/variety).
- The cutting time or timeline of the crop (i.e. at what time(s) the crop was cut). The cutting time/timeline may comprise date and/or time data. For example, the cutting time/timeline may comprise [day,month,year] data. The data may comprise a series of date/time data points, where cutting is performed multiple time. Cutting data may be obtained from one or more: harvesting/cutting equipment having tracking or monitoring capabilities; local registries or databases; or manual input.
- The cutting frequency of the crop. The frequency may be determined over a specific time period (e.g. week, month, year etc.). Typically, the cutting frequency is the number of times the grass is cut in a calendar year or growing season. Cutting data may be obtained from one or more: harvesting/cutting equipment having tracking or monitoring capabilities; local registries or databases; or manual input.
- The processing applied to the crop/fodder. This may comprise the type of processing: for example: raking; tedding; baling; chopping; drying; dehydration; compaction; and wrapping. The processing parameter may further comprise one or more of: the (time) length of a given processing step; a drying rate; a moisture profile (e.g. starting and/or finishing moisture level of the crop); start and/or finish density of the fodder; a wrapping material. Processing data may be obtained from one or more: harvesting/cutting equipment having tracking or monitoring capabilities; local registries or databases; or manual input.
- The farming technique or other agricultural processing of the crop. The farming technique may indicate practices relating one or more of: seeding (e.g. seeding date, seeding coverage, seeding density); fertilisation (e.g. type, amount, coverage thereof); or soil manipulation (e.g. amount or type of pH raising/lowering agents). Data may be obtained from one or more: harvesting/cutting equipment having tracking or monitoring capabilities; local registries or databases; or manual input.
- The soil composition in which the crop is grown. The soil composition may comprise one or more: organic content; mineral content; or water content. Data may be obtained from, one or more of: authority (e.g. government or government agency) registries; national databases; laboratory analysis; or manual input.
- The condition of the fodder after processing. The data may comprise one or more of: temperature; moisture level; pH; form factor; or density of the fodder. Measurements may be for a single instance or may be made over a period of time (i.e. multiple time spaced measurements are made). Data may be obtained from one or more of: satellite imagery; aerial photography (e.g. via drones); harvesting/cutting equipment having tracking or monitoring capabilities; local registries or databases; local analysis (e.g. with portable sensors); laboratory analysis; or manual input.
- Weather conditions. The weather conditions may be indicative of the weather conditions during growing of the crop, harvesting of the crop; processing of the crop; after processing of the crop; and/or during storage of the fodder. Data may be obtained across one or more time period of the crop growing/processing/storage stage and/or may be continuously recorded. The weather conditions may comprise one or more of: temperature; precipitation amount (e.g. rainfall); humidity; wind direction; wind speed; atmospheric pressure; growing degree days; or dew points. Weather data may be obtained from one or more of: satellite imaging or data; local or national weather databases; proprietary databases; local registries; local sensor measurements; or human inputs.

Sets of data for one or more of the above variables are then associated with known compositions of fodder in a training step 12. The relationship between the specific composition of the foodstuff and the above variables can then be correlated. The composition of the fodder can be determined according to a plurality of output variables. Thus, each output variable can be estimated for a given set of input variables accordingly. In general, the output variables comprise a nutritional value of the fodder. The composition variables may comprise one or more of:
- Protein content. For example, crude proteins (CP) and/or specific amino acid levels.
- Carbohydrate content. For example, non-fibre carbohydrates (NFC); water-soluble carbohydrates (WSC); ether-soluble carbohydrates (ESC); and/or specific sugar contents (e.g. fructose, sucrose, fructan).
- Fat content.
- Fibre content. For example, neutral detergent fibres (aNDF); acid detergent fibres (ADF); and/or acid detergent lignin (ADL).
- Other broad indicators of nutritional value. For example, total digestible nutrients (TDN); relative feed value (RFV), relative feed quality (RFQ); and/or calorific content.
- Moisture content and/ or dry content. For example, the volume/weight percentage moisture.
- Mineral/vitamin content. For example, one or more of: Calcium (Ca); Phosphorus (P); Sodium (Na); Chloride (Cl); Magnesium (Mg); Potassium (K); Sulphur (S) Cobalt (Co); Copper (Cu); Fluoride (F); Iron (Fe); Manganese (Mn); Molybdenum (Mo); Selenium (Se); or Zinc (Zn)

The composition of the fodder may be represented by: ${Q}_{n} = F \left({L}_{n}, {S}_{n}, {C}_{n}, {G}_{n}, {P}_{n}, {A}_{n}, {B}_{n}, {M}_{n}, {H}_{n}\right) + ε$

Where Qₙ represents the fodder composition for a given variable or set of variables; F is a function of the input variables as described above; and ε represents an arbitrary constant. It can be appreciated the function F may non-linear or otherwise non-trivial.

Lₙ represents the geographical (location) data; Sₙ represents the species of the crop; Cₙ represents the cutting timeline of the crop; Gₙ represents the cutting frequency; Pₙ represents the processing parameters; Aₙ represents the farming practices; Bₙ represents the soil composition; Mₙ represents the fodder conditions; and Hₙ is the weather condition. The input and/or output variables are typically provided as matrix of parameters (in particular, as many of the variables may be multidimensional).

It can be understood that the composition may have a linear relationship with some input variables, however, typically the input variables interact with one another, and thus the composition comprises a sum of linear factors and interactive (i.e. non-linear) factors between the variables. The composition may therefore be expressed by: $\begin{matrix}{Q}_{n} = {β}_{1} . {L}_{n} + {β}_{2} . {S}_{n} + {β}_{3} . {C}_{n} + {β}_{4} . {G}_{n} + {β}_{5} . {P}_{n} + {β}_{6} . {A}_{n} + {β}_{7} . {B}_{n} + {β}_{8} . {M}_{n} + \\ {β}_{9} . {H}_{n} + {β}_{10} . {L}_{n} . {S}_{n} + {β}_{11} . {C}_{n} . {G}_{n} + {β}_{12} . {P}_{n} . {A}_{n} + \sum {β}_{n} . Interactive factors + ε\end{matrix}$

Where βₙ are numerical constants and Interactive factors are single or multiple combinations of the matrixes Lₙ, Sₙ, Cₙ, Gₙ, Pₙ, Aₙ, Bₙ, Mₙ and Hₙ. Qₙ may be represented as a list of strings, a table of numerical values. Qₙ may be displayed on paper or digital format or any other means.

Data sets for the input variables associated with the known composition values are input into the computational model. The model may comprise a neural network, machine learning model or other multi-variable regression model. Typically, a large number of data sets are input into the system. The known composition data may be obtained from any suitable source, for example, one or more of: databases (e.g. government or proprietary databases); or manual input (for example, the user can train their own system). Composition data may be obtained from a plurality of sources. For example, each user of the system may input their own data. Composition data may therefore be collectively obtained (i.e. crowd-sourcing). The system is trained until a suitable degree of accuracy can be obtained.

Each data set need not contain data for each input variable. Similarly, each composition value need not contain data for each output variable. With a large number of samples, the computational model may be constructed even when datasets are "incomplete".

Once the system has been trained, the user can input specific values for their foodstuff for the input variables in step 14. This may be input in any suitable fashion. Data may manually input and/or retrieved from remote sources, as discussed above.

In step 16, the model uses the input variable values to estimate the composition of the fodder. The user may use the model to estimate any or all of the composition variables discussed above. For example, the user may use the system to only estimate the fibre content of the fodder, or the fibre and carbohydrate content etc. The user may obtain values for the input variables from the sources discussed above. Where the user has processed the fodder themselves, then typically the input variable values may be manually input. With the estimated composition of the fodder, the user can adjust the feeding regime of the animals accordingly.

The present system mitigates the need to use laboratory testing to determine composition. However, in some embodiments, the laboratory testing may still be used to verify the estimated composition. The laboratory testing may then be input into the computational model to provide further training data. The system may therefore be continually trained. Nevertheless, the user may continue to use less laboratory testing as the accuracy of the model increases. If many users are using the system, then even a small sample of laboratory testing may provide large volumes of data. The training data is therefore pooled over a large number of users (e.g. the system gains an economy of scale).

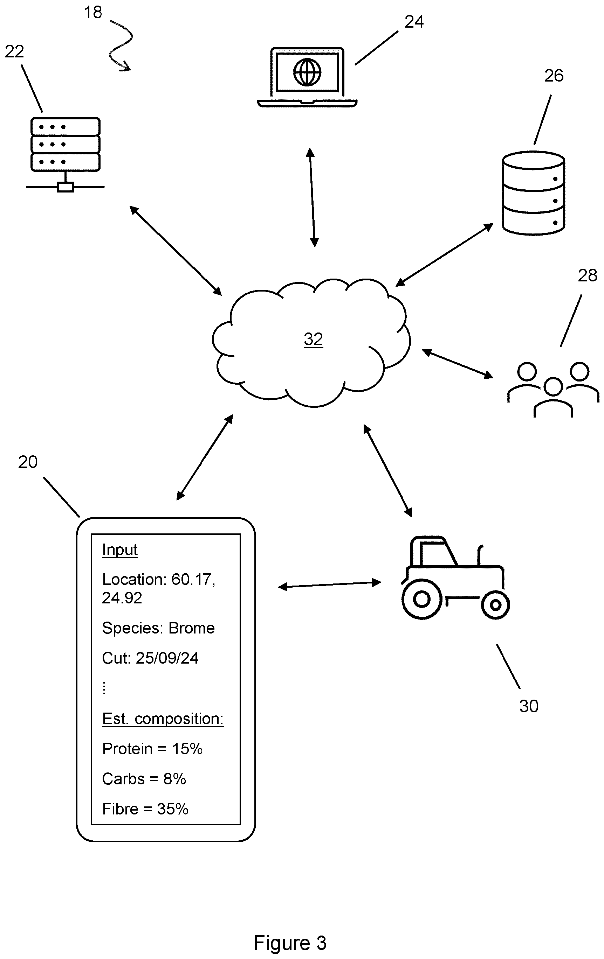

The system 18 is shown schematically in figure 3. A user device 20 allows the user to input values for the input variables. The user can then view the compositional data accordingly. The user device 20 may comprise any suitable device, for example, inter alia: a mobile (cellular) device; a tablet computer; a laptop computer; a desktop computer; server; single-board computer (SBC); server; or other conventional computer. The user device 20 may comprise an application or other software to allow input of data and/or to receive composition estimates. The user device 20 allows the user to manually input data. The user device 20 may allow the user to import and/or retrieve data from remote sources (e.g. weather data).

Data processing (e.g. training of the model and/or estimating of the composition of the fodder) may be provided by a remote server 22. This allows operation of the system to be centralised and mitigates the need for processing on the user devices. Input variable values may be transmitted to the remote server 22 and the estimated composition values are then transmitted back to the user device 20. The remote server 22 may comprise any conventional computing device, for example, inter alia: server; cloud computing service; supercomputer or computer cluster. Processing may be performed over one or more device. In other embodiments the data processing is performed on the user device 20.

The remote server 22 and/or user device 20 may be operatively connected to other data sources, such as internet resources 24, databases 26 (e.g. government databases), other users 28 and/or agricultural machinery 30. The above entities may be connected through "the cloud" 32 (e.g. the internet). The remote server 22 and/or user device 20 may download data from the further sources to provide training for the model and/or to provide input values for the composition estimate. The system may be configured to interact with an API or the like provided on the external databases. The agricultural machinery 30 may be operatively connected directly to the user device 20. The user device 20 and/or other users 28 may transmit data to the remote server 22 and/or one another (e.g. peer-to-peer), for example, training data.

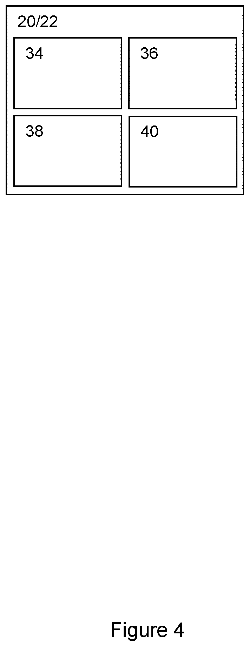

Figure 4 shows a schematic of the processing apparatus 20/22 configured to provide the computational model. The apparatus comprises a processor 34. The processor may comprise one or more of: logic components; standard integrated circuits; application-specific integrated circuits (ASIC); system-on-a-chip (SoC); application-specific standard products (ASSP); microprocessors; microcontrollers; digital signal processors; special-purpose computer chips; field-programmable gate arrays (FPGA); and other suitable electronics structures. The apparatus may comprise volatile memory 36 (e.g. RAM). The apparatus may comprise non-volatile memory (e.g. a hard-drive, SSD, read-only memory etc.). The processor may comprise a graphical processing unit (GPU). The GPU may be integral or form a separate unit.

The apparatus may comprise a communication interface 38. The communication interface may comprise a wired or wireless interface (e.g. Wifi or mobile/cellular interface). It can be appreciated that the exact form of the hardware and/or software used to implement the present system is not pertinent to the invention at hand.

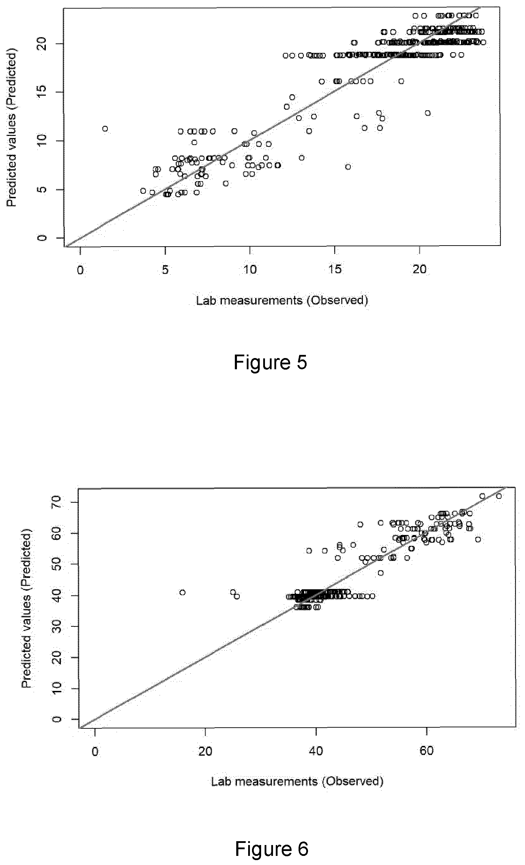

Figure 5 shows the predicted compositional crude protein (CP) values using the computational model and the compositional crude protein (CP) values determined using laboratory testing. Figure 6, likewise shows the predicted compositional neutral detergent fibres (aNDF) values using the computational model and the compositional neutral detergent fibres (aNDF) values determined using laboratory testing. It can be seen from the figures that computational model provides a good degree of accuracy and there is a good correlation between estimated values from the computational model and the laboratory testing. Comparison between estimated values from the computational model and the laboratory testing may be used to indicate a certain degree of accuracy to the user (e.g. as an error range etc.).

## Claims

1. A method of estimating a composition of a foodstuff comprising:
providing a plurality of input variables, the input variables relating to the growing and/or processing conditions of the foodstuff;
providing a computational model comprising data relating to the input variables and data relating to a known composition of a foodstuff associated with the input variables; and
receiving a value for a plurality of input variables and estimating the composition of the foodstuff in accordance with said input variable values using said computational model.

2. A method according to claim 1, comprising receiving data relating to the input variables and data relating to the known composition of a foodstuff associated with the input variables to train said computational model.

3. A method according to any preceding claim, where the growing and/or processing conditions comprises one or more of: a geographic location of foodstuff during growth thereof; a coverage rate of the foodstuff; a species or variety of the foodstuff; a cutting time or timeline of the foodstuff; a cutting frequency of the foodstuff; a processing state of the foodstuff; farming practices for the foodstuff; soil composition in which the foodstuff is grown; condition of the foodstuff after processing; or weather condition curing processing or growth of the foodstuff.

4. A method according to any preceding claim, where the foodstuff composition comprises a nutritional value of the foodstuff.

5. A method according to any preceding claim, where the nutritional value of the foodstuff comprises one or more of: a protein content; a carbohydrate content; fibre content; moisture content; or mineral/vitamin content.

6. A method according to any preceding claim, where the foodstuff comprises a crop or processed product thereof, and the crop comprises one or more of: grass; legumes; or cereal crops.

7. A method according to any preceding claim, where the composition of the foodstuff is estimated after harvest and/or subsequent processing thereof.

8. A method according to any preceding claim, where foodstuff comprises one or more of: hay; silage; haylage; straw; sprouted grains; legumes; or pellets of said crops.

9. A method according to any preceding claim, where the computational model comprises a neural network or machine learning model.

10. A method according to any preceding claim, where a laboratory composition value for a given set of values for the input variables is input into the computational model and compared to the corresponding estimated composition data associated with said set of values for the input variables to further train said computational model.

11. A method according to any preceding claim, where input variable data and/or values are received from a remote database.

12. A method according to any preceding claim, where input variable data and/or values are received from an agricultural processing and/or harvesting machine.

13. A method according to any preceding claim, comprising a plurality of users, each user being able to estimate the composition and where a common computational model is used to provide said estimate the users.

14. A computer program or computer readable medium comprising program instructions which, when executed by the computer, cause the computer to carry out a computer process implementing the method according to any preceding claim.

15. A computer system (20,22) configured to estimate a composition of a foodstuff comprising a controller (34) configured to:
provide a plurality of input variables, the input variables relating to the growing and/or processing conditions of the foodstuff;
provide a computational model comprising data relating to the input variables and data relating to the known composition of a foodstuff associated with the input variables; and
receive a value for a plurality of input variables and estimate the composition of the foodstuff in accordance with said input variable values using said computational model.
